# EUROPEAN PATENT SPECIFICATION

(11) **EP 1 744 672 B1**
(45) Date of publication and mention of the grant of the patent: **07.03.2012**
(21) Application number: 04726168.0
(22) Date of filing: 07.04.2004
(51) Int. Cl.: A61B 5/087, A61B 5/091

(54) **DEVICE FOR ASSESSMENT OF INHALATION FLOW AND VOLUME**
VORRICHTUNG ZUR BEURTEILUNG DES INHALATIONSFLUSSES UND DES VOLUMENS
DISPOSITIF PERMETTANT DE MESURER LE FLUX ET LE VOLUME D'INHALATION

(43) Date of publication of application: 24.01.2007
(73) Proprietor: Medinet S.p.A., 20154 Milano (IT)
(72) Inventor: APOLET, Josef, Berek, I-20146 Milano (IT); GIARDINI, Mario, Ettore, I-27051 Cava Manara (IT)
(74) Representative: Gervasi, Gemma
(86) International application number: PCT/EP2004/050467
(87) International publication number: WO 2005/096933

(56) References cited:
- WO-A-98/36687
- DE-U- 29 916 407
- US-A- 4 114 607
- US-A- 4 183 361

## Description

### FIELD OF THE INVENTION

The field of the invention concerns devices for respiratory exercise.

### PRIOR ART

Devices that estimate or measure inhalation or exhalation flow speed and lung capacity are known medical instruments. Several types are currently available on the market; as shown e.g. by WO-A-98/36687.

They generally consist of sections that permit determination of inhalation and/or exhalation speed and flow. Said measurements are performed by means of assessment, if necessary quantitative, of the rotation speed of rotating turbines, or by measuring the drop in pressure at the ends of narrowed pipe sections, or by measuring the variations in volume of pre-defined chambers.

In particular, flow measuring devices are described in literature and are available on the market that use the movement of an obstacle in a chamber with tapered section. In said devices, the air passes from the bottom to the top of a truncated cone vertical chamber with the largest end at the top in which a mobile obstacle is present, thus moving said obstacle upwards. The position of equilibrium of the obstacle depends on the flow speed and permits measurement of it. Similarly, respiratory volume measuring devices are furthermore described and used in which the air is inhaled from a cylindrical chamber wherein a sealed piston runs. The volume inhaled and/or exhaled is equal or proportional to the movement of said piston.

These devices, often very complex, are generally used only as measuring devices and only rarely as devices for breathing exercises.

Devices that measure the inhaled volume by means of mobile obstacles (such as pistons) which, instead of being a perfect seal, allow air leaking towards a suitable chamber, at present do not exist on the market. Furthermore, devices which, in addition to measuring lung capacity and respiratory flow values, enable the doctor to establish respiratory objectives and the patient to ascertain if and when they are achieved do not currently exist on the market.

### SUMMARY OF THE INVENTION

The present invention concerns a device for assessing the actual inhalation flow and inhalation volume of a patient; the device comprises a first chamber in the shape of an upright cylinder containing a first mobile obstacle for estimation of the total inhalation volume; the first chamber communicates, via a communication tube, with a second chamber the cross sections of which decrease downwards and containing a second mobile obstacle for estimation of the inhalation flow, where said first and second mobile obstacles are free to move vertically in their respective chambers and where said chambers and the communication tube have an open lower section that provides communication with the outside.

Preferably the first chamber has the shape of a vertical cylinder, and the second chamber has the shape of a vertical frustum of cone with the largest end at its top. Preferably the first chamber consists of or comprises a rigid tube; preferably the first and second chambers communicate with the outside by means of sections at the base of the tubes (1, 2) and (6), preferably provided with the filters (7). The tube (6) can also be fitted with a hose (5) with mouthpiece.

Each chamber (1) and (2) can contains an obstacle, respectively (3) and (4), preferably spherical, free to move inside the chambers; the first mobile obstacle (4) in the first chamber (2) does not provide a perfect seal with said chamber.

Preferably one or more mobile indicators (9) and (10) are also preferably present at least on the tube (2).

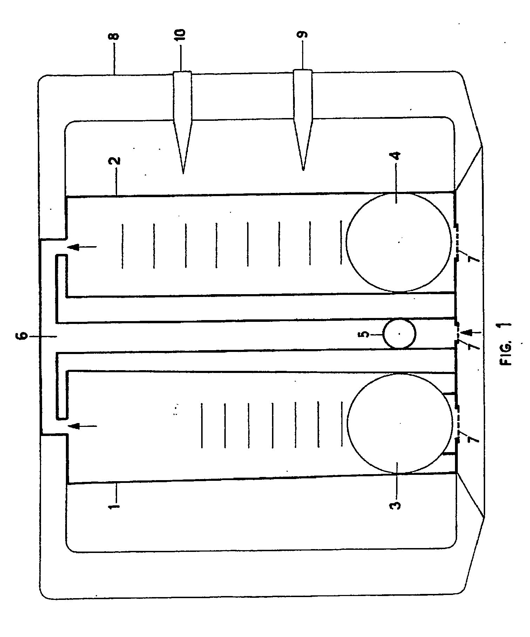

A particular embodiment of said device is constituted by the apparatus described in figure 1.

The apparatus, via the free movement of the obstacles 3 and 4 inside the chambers 1 and 2, as a result of the inhalation, provides an estimate of the inhalation flow (volume/time) and an estimate of the total inhalation volume: as such it therefore functions as a spirometer. The value of the estimate is indicated by the height reached by the mobile obstacles 3 and 4 inside the chambers 1 and 2.

The apparatus also has a stimulation function: once the operator has assessed the patient's total lung flow and capacity, by observing or shifting the mobile indicators 9 and 10 according to the required clinical protocol, he can establish one or more objectives and/or visually ascertain that they have been achieved. The objectives are established by assessing the inhalation results obtained and the general conditions of the patient: they can be intermediate or final objectives. On the back of the device, consisting of a transparent screen, preferably with rough finish, objectives, results and/or any additional information (for example any improvements achieved over time) can be recorded, for example by marking with a felt pen, pencil or by applying adhesive labels.

### DESCRIPTION OF THE FIGURE

Figure 1. Vertical section of the respiratory stimulation device.
(1): rigid graduated tube in the shape of a vertical truncated cone with largest end at the top for identifying the inhalation flow containing a spherical obstacle 3, of diameter substantially equal to or slightly smaller than the diameter of the smallest end of the truncated cone;
(2): rigid graduated tube in the shape of a vertical cylinder for estimation of the total inhalation volume, containing a spherical obstacle 4 of diameter slightly smaller than the diameter of the cylinder base;
(5): a pipe, preferably a hose, and if necessary corrugated, fitted with a mouthpiece;
(6): communication tube between tubes 1 and 2 and pipe 5;
(7): filtering means;
(8): handle;
(9) and 10: mobile indicators.

### DETAILED DESCRIPTION OF THE INVENTION

The present invention concerns a device comprising a second chamber 1 for assessment of the inhalation flow and a first chamber 2 for assessment of the inhalation volume, where said chambers communicate with each other at the top by means of a communication tube 6 and with the outside by means of sections at the base of 1, 2 and 6.

The device provides an estimate of the inhalation flow (for example by means of volume/time or weight/time values or by means of arbitrary values) and an estimate of the total inhalation volume: as such it therefore functions as a spirometer. The device also has a stimulation function: once the patient's lung flow speed and capacity have been determined, on the basis of one or two inhalations, it also permits one or more objectives to be established according to the patient's general conditions and/or recovery capacities.

The objective can be established after reading the total flow speed and capacity values provided by the spirometer and according to the general conditions of the patient, as judged appropriate by the doctor.

The apparatus shown in figure 1 is a particular embodiment of said device.

In the device of the invention the second chamber consists essentially of a rigid tube 1 in the shape of a vertical truncated cone with larger end at the top for estimation of the inhalation flow and the first chamber consists of a rigid tube 2 in the shape of a vertical cylinder for estimation of the total inhalation volume, each one containing an obstacle, preferably spherical, 3 and 4 free to move inside the tubes. In the chamber 2 the mobile obstacle 4 does not provide a perfect seal with the chamber, as in chamber 1; more generally the first chamber 2 can be an upright cylinder or tubular cavity, and the second chamber 1 has cross sections decreasing downwards.

The tubes 1, 2 and 6 communicate with the outside by means of sections at the base, preferably provided with filters 7 and communicate with each other at the level of the upper base via the communication tube 6.

Externally, on the side opposite the side with the pipe 5 fitted with mouthpiece, the device is preferably closed completely and preferably has a rough surface which is used, for example, for recording information concerning the patient or the last date the device was used, by means of application of permanent or semi-permanent wording or with adhesive labels.

The communication tube 6 is fitted with a pipe 5, preferably a hose or corrugated, provided with a mouthpiece, preferably removable.

All the tubes 1, 2 and 6 have a diameter such that there is no resistance to passage of the air harmful for the patient.

The apparatus is characterised by the presence of a slight play between the tubes 1 and 2 and the respective obstacles 3 and 4 due to the difference in diameter, since the section of the obstacles is smaller than the base section of the tubes. In addition to the above-mentioned characteristics, the apparatus preferably features one or more indicators 9 and 10 or devices for displaying, signalling or highlighting on the tubes 1 and 2, or even only on tube 2, results and objectives established by the doctor and/or achieved by the patient.

Other movable indicators can optionally record flow and/or volume estimates or measurements or any other flow and/or volume references.

The indicators can be fitted on the tubes 1 and 2, as for example when they are movable rings, or they can be fitted outside the tubes 1 and 2, preferably on a handle 8, as in the embodiment described in figure 1 or on both the handles described in figure 1.

The rigid tubes 1 and 2 are sufficiently transparent or translucent to permit display of the respective obstacles 3 and 4. Both tube 1 and tube 2 are preferably graduated according to the flow and volume respectively, in the corresponding units of measurement or with arbitrary units of measurement.

Measurement of lung flow and volume is obtained by free movement of the obstacles 3 and 4 in the rigid tubes 1 and 2, where said movement is obtained by inhalation which determines a flow of air at the inlet through the sections at the base of the tubes 1, 2 and 6. The obstacles 3 and 4, free to move vertically inside the rigid tubes 1 and 2, are preferably spherical with diameter slightly smaller than the diameter of the smaller end of the truncated cone and the cylinder, so that between tubes 1 and 2 and mobile obstacles 3 and 4 there is an air leak, pre-defined in order not to affect the measurement, at the same time minimising the effects of friction of the tubes on the obstacles.

In the tube 1 and 2 the mobile obstacles 3 and 4 are preferably resting on a means or support designed to maintain said obstacles raised from the lower base of the tube to prevent adhesion of the obstacles, due to Venturi effect, to the sections of the base from which the air enters.

The tubes 1 2 and 6 are open also at the bottom by means of sections that connect them to the outside and which permit the air inflow. The openings on the bottom (sections) are fitted preferably with filters 7, optionally replaceable, to prevent the entry of dust or debris inside the apparatus and thus prevent possible obstructions to the movement of the obstacles 3 and 4 in the tubes 1 and 2 by particulate material or other coming from the outside.

The stimulation apparatus is used as follows:
- the patient inhales into the apparatus by appropriately positioning the mouthpiece fitted at the end of the hose 5 in the mouth;
- the inhalation draws air by means of the tube 6 via the rigid tubes 1 and 2; said air enters the apparatus from the inlets of 1, 2, and 6, and is filtered by the filters 7;
- in tube 1 the air raises the obstacle 3 to a level proportional to the value of the inhalation speed of the respiratory flow and in tube 2, it raises the obstacle 4 to a level proportional to the total volume of air inhaled;
- a medical operator uses the flow and volume values determined by observation of the height reached by the obstacles 3 and 4 in the tubes 1 and 2, optionally also observes the time the mobile obstacles 3 and 4 remain in the tubes 1 and 2, and then sets or instructs the patient to set the indicators 9 and 10 as objectives, according to the required clinical protocols.

The apparatus of the invention furthermore comprises preferably at least one handle 8 which can be used by the patient to appropriately position the apparatus during inhalation. Preferably there are two handles in a position external and lateral to the tubes 1 and 2, to permit use of the device by both right and left-handed patients.

In the embodiment described in figure 1 the handle 8 is provided with the sliding indicators 9 and 10 which, being positioned near to or on the tube 2, estimate the volume values. The lower indicator 9 can be distinguished by colour, code or other from the upper one 10, the lower indicator signalling the result obtained by the patient and the upper one the objective to be achieved, established by the doctor.

The sliding indicators can also be positioned directly on the cylinder 2. Indicators with the same function can also be provided on the tube 1 indicating in this case respiratory flow results and objectives.

The main characteristic of the device developed is its simplicity of operation. From the user's point of view, it should also be pointed out that immediate visualisation of the results obtained in breathing exercises stimulates use of the apparatus in order to achieve the set objectives.

The device can also be easily handled and is inexpensive: as it is not based on electric or electronic devices it can be used anywhere without electrical connections or batteries. The apparatus does not contain hydraulic seal elements or elements depending on friction, the operation of which can be altered more easily than a device such as the one developed.

Easy visualisation of the lung flow and capacity results obtained by inhalation and comparison between the result obtained and the objective established results in stimulation of respiratory exercise and immediate visualisation of any improvements obtained.

Due to the stimulation function, it is particularly useful for patients with respiratory problems, associated with the possibility of improving respiratory performance via exercise of the inhalation function.

## Claims

1. Device for assessing inhalation flow and volume comprising a first chamber (2) in the shape of an upright cylinder containing a first mobile obstacle (4), wherein said first chamber communicates, via a communication tube (6), with a second chamber (1) the cross sections of which decrease downwards and containing a second mobile obstacle (3), where said first and second mobile obstacles (3, 4) are free to move vertically in their respective chambers (1) and (2), **characterized in that** said chambers (1, 2) and the communication tube (6) have an open lower section that provides communication with the outside and **in that** said first mobile obstacle (4) does not provide a perfect seal in said first chamber (2).

2. Device according to claim 1, wherein said first chamber (2) has the shape of a vertical cylinder, said second chamber (1) has the shape of a vertical frustum of cone with the largest end at its top.

3. Device according to claim 1 or 2 wherein at least one of said mobile obstacles are spherical.

4. Device according to one or more claims 1-3 wherein the first chamber (2) has at least one first mobile indicator (9, 10).

5. Device according to one or more claims 1-4 wherein the second chamber (1) has at least one second mobile indicator (9, 10).

6. Device according to claims 4 and 5 wherein at least one of said indicators (9, 10) is sliding and positioned on the tube corresponding to the first or second chamber (1, 2) respectively.

7. Device according to claims 4 and 5 wherein at least one of said indicators (9, 10) is positioned outside the tubes corresponding to said first or second chambers (1, 2) respectively.

8. Device according to one or more claims 1-7, comprising at least one handle (8).

9. Device according to claims 7 and 8, wherein the indicators positioned outside the first chamber (2) are positioned on the handle (8).

10. Device according to one or more claims 1-9, wherein the communication tube (6) is connected to a pipe (5) fitted with a mouthpiece or other means suitable for permitting inhalation into the device.

11. Device according to claim 10 wherein said mouthpiece or other means can be removed and changed.

12. Device according to one or more claims 1-10, wherein the pipe (5) is flexible.

13. Device according to one or more claims 1-12, wherein at least one of said first or second chamber (1, 2) are graduated.

14. Device according to claim 13 wherein the second chamber (1) is graduated with flow measurements and the first chamber (2) with volume measurements.

15. Device according to claim 13 wherein at least one of said first or second chambers (1, 2) are graduated with arbitrary units.

16. Device according to one or more claims 1-15, wherein in the second chamber (1) the second obstacle (3) rests on a means or support means suitable for maintaining said second obstacle raised from the section on the lower base of the second chamber.

17. Device according to one or more claims 1-16, wherein the first obstacle (4) in the first chamber (2) rests on a means or support means suitable for maintaining said first obstacle raised from the section on the lower base of the chamber.

18. Device according to one or more claims 1-17, wherein the sections on the bottom of the chambers (1, 2) and the communication tube (6) at the base are provided with filters (7) or means suitable for preventing the entry of dust or debris inside the apparatus.

19. Device according to claim 18 wherein said filters (7) can be changed.

20. Device according to one or more claims 1-19, wherein said device is preferably closed and preferably comprises a rough surface on the side opposite the one with the pipe (5).

## Patentansprüche

1. Vorrichtung zur Bewertung des Volumenstromes der Inhalation und des Atemvolumens, welche eine erste Kammer (2) in Gestalt eines aufrechten Zylinders umfasst, der ein erstes bewegliches Hindernis (4) enthält, wobei die genannte erste Kammer über ein Verbindungsrohr (6) mit einer zweiten Kammer (1) in Verbindung steht, deren Querschnitte nach unten hin abnehmen und welche ein zweites bewegliches Hindernis (3) enthält, wobei das genannte erste und das genannte zweite bewegliche Hindernis (4, 3) sich in ihren jeweiligen Kammern (1) bzw. (2) in der vertikalen Richtung frei bewegen können, **dadurch gekennzeichnet, dass** die genannten Kammern (1, 2) und das Verbindungsrohr (6) einen offenen unteren Querschnitt aufweisen, welcher die Verbindung nach außen herstellt, und dadurch, dass das genannte erste bewegliche Hindernis keine vollständige Dichtung in der genannten ersten Kammer (2) bildet.

2. Vorrichtung nach Anspruch 1, bei welcher die genannte erste Kammer (2) die Gestalt eines vertikalen Zylinders hat und die genannte zweite Kammer (1) die Gestalt eines vertikalen Kegelstumpfes hat mit der großen Fläche oben.

3. Vorrichtung nach Anspruch 1 oder 2, bei welcher mindestens eines der genannten beweglichen Hindernisse kugelförmig ist.

4. Vorrichtung nach einem oder mehreren der Ansprüche 1 bis 3, bei welcher die erste Kammer (2) mindestens eine erste bewegliche Anzeigevorrichtung (9, 10) aufweist.

5. Vorrichtung nach einem oder mehreren der Ansprüche 1 bis 4, bei welcher die zweite Kammer (1) mindestens eine zweite bewegliche Anzeigevorrichtung (9, 10) aufweist.

6. Vorrichtung nach den Ansprüchen 4 und 5, bei welcher mindestens eine der genannten Anzeigevorrichtungen (9, 10) gleitend ist und an dem Rohr positioniert ist, welches der ersten bzw. der zweiten Kammer (2, 1) entspricht.

7. Vorrichtung nach den Ansprüchen 4 und 5, bei welcher mindestens eine der genannten Anzeigevorrichtungen (9, 10) außerhalb der Rohre positioniert ist, welche der ersten bzw. der zweiten Kammer (2, 1) entsprechen.

8. Vorrichtung nach einem oder mehreren der Ansprüche 1 bis 7, welche mindestens einen Handgriff (8) umfasst.

9. Vorrichtung nach den Ansprüchen 7 und 8, bei welcher die außerhalb der ersten Kammer (2) positionierten Anzeigevorrichtungen am Handgriff (8) positioniert sind.

10. Vorrichtung nach einem oder mehreren der Ansprüche 1 bis 9, bei welcher das Verbindungsrohr (6) mit einem Schlauch (5) verbunden ist, welcher mit einem Mundstück oder einem anderen Mittel verbunden ist, welches geeignet ist, die Inhalation in die Vorrichtung zu ermöglichen.

11. Vorrichtung nach Anspruch 10, bei welcher das genannte Mundstück oder andere Mittel entfernt und ausgetauscht werden kann.

12. Vorrichtung nach einem oder mehreren der Ansprüche 1 bis 10, bei welcher der Schlauch (5) flexibel ist.

13. Vorrichtung nach einem oder mehreren der Ansprüche 1 bis 12, bei welcher mindestens eine der genannten ersten und zweiten Kammer (1, 2) graduiert ist.

14. Vorrichtung nach Anspruch 13, bei welcher die zweite Kammer (1) hinsichtlich der Volumenstrommessung und die erste Kammer (2) hinsichtlich der Volumenmessung graduiert ist.

15. Vorrichtung nach Anspruch 13, bei welcher mindestens eine der genannten ersten oder zweiten Kammer (2, 1) in willkürlichen Einheiten graduiert ist.

16. Vorrichtung nach einem oder mehreren der Ansprüche 1 bis 15, bei welcher in der zweiten Kammer (1) das zweite Hindernis (3) auf einem Mittel oder einer Stützvorrichtung ruht, welche geeignet ist, das genannte zweite Hindernis vom Querschnitt an der Unterseite der zweiten Kammer im angehobenen Zustand zu halten.

17. Vorrichtung nach einem oder mehreren der Ansprüche 1 bis 16, bei welcher das erste Hindernis (4) in der ersten Kammer (2) auf einem Mittel oder einer Stützvorrichtung ruht, welche geeignet ist, das genannte erste Hindernis vom Querschnitt an der Unterseite der Kammer im angehobenen Zustand zu halten.

18. Vorrichtung nach einem oder mehreren der Ansprüche 1 bis 17, bei welcher die Querschnitte am Boden der Kammern (1, 2) und das Verbindungsrohr (6) am Unterteil mit Filtern (7) oder mit anderen Mitteln ausgestattet sind, welche geeignet sind, das Eindringen von Staub oder Schmutzstoffen in das Innere der Apparatur zu verhindern.

19. Vorrichtung nach Anspruch 18, bei welcher die genannten Filter (7) ausgetauscht werden können.

20. Vorrichtung nach einem oder mehreren der Ansprüche 1 bis 19, bei welcher die genannte Vorrichtung vorzugsweise geschlossen ist und auf der Seite, welche der Seite mit dem Rohr (5) gegenüber liegt, vorzugsweise eine raue Oberfläche umfasst.

## Revendications

1. Dispositif pour mesurer le flux et le volume d'inhalation comprenant une première chambre (2) sous la forme d'un cylindre vertical contenant un premier obstacle mobile (4) où ladite première chambre communique, par un tube de communication (6), avec une deuxième chambre (1) dont les sections transversales diminuent vers le bas et contenant un deuxième obstacle mobile (3), où lesdits premier et deuxième obstacles mobiles (3, 4) sont libres de se déplacer verticalement dans leurs chambres respectives (1) et (2), **caractérisé en ce que** lesdites chambres (1) et (2) et le tube de communication (6) ont une section inférieure ouverte qui établit une communication avec l'extérieur, et **en ce que** ledit premier obstacle mobile (4) ne réalise pas un joint parfait dans ladite première chambre (2).

2. Dispositif selon la revendication 1, dans lequel ladite première chambre (2) a la forme d'un cylindre vertical, ladite deuxième chambre (1) a la forme d'un cône tronqué vertical avec l'extrémité la plus grande en haut.

3. Dispositif selon la revendication 1 ou 2, dans lequel au moins un desdits obstacles mobiles est sphérique.

4. Dispositif selon l'une ou plusieurs des revendications 1 à 3, dans lequel la première chambre (2) possède au moins un premier indicateur mobile (9, 10).

5. Dispositif selon l'une ou plusieurs des revendications 1 à 4, dans lequel la deuxième chambre (1) possède au moins un deuxième indicateur mobile (9, 10).

6. Dispositif selon les revendications 4 et 5, dans lequel au moins un desdits indicateurs (9, 10) coulisse et est positionné sur le tube correspondant à la première ou deuxième chambre (1, 2) respectivement.

7. Dispositif selon les revendications 4 et 5, dans lequel au moins un desdits indicateurs (9, 10) est positionné à l'extérieur des tubes correspondant auxdites première et deuxième chambres (1, 2), respectivement.

8. Dispositif selon une ou plusieurs des revendications 1 à 7, comprenant au moins une poignée (8).

9. Dispositif selon les revendications 7 et 8, dans lequel les indicateurs positionnés à l'extérieur de la première chambre (2) sont positionnés sur la poignée (8).

10. Dispositif selon une ou plusieurs des revendications 1 à 9, dans lequel le tube de communication (6) est relié à un tuyau (5) pourvu d'un embout ou autre moyen apte à permettre l'inhalation dans le dispositif.

11. Dispositif selon la revendication 10, dans lequel ledit embout ou autre moyen peut être retiré et changé.

12. Dispositif selon une ou plusieurs des revendications 1 à 10, dans lequel le tuyau (5) est flexible.

13. Dispositif selon l'une ou plusieurs des revendications 1 à 12, dans lequel au moins une desdites première ou deuxième chambre (1, 2) est graduée.

14. Dispositif selon la revendication 13, dans lequel la deuxième chambre (1) est graduée avec des mesures d'écoulement, et la première chambre (2) avec des mesures de volume.

15. Dispositif selon la revendication 13, dans lequel au moins une de ladite première ou deuxième chambre (1, 2) est graduée avec des unités arbitraires.

16. Dispositif selon l'une ou plusieurs des revendications 1 à 15, dans lequel dans la deuxième chambre (1), le deuxième obstacle (3) repose sur un moyen ou moyen de support approprié pour maintenir ledit deuxième obstacle relevé de la section sur la base inférieure de la deuxième chambre.

17. Dispositif selon l'une ou plusieurs des revendications 1 à 16, dans lequel le premier obstacle (4) dans la première chambre (2) repose sur un moyen ou moyen de support apte à maintenir ledit premier obstacle relevé de la section sur la base inférieure de la chambre.

18. Dispositif selon l'une ou plusieurs des revendications 1 à 17, dans lequel les sections sur le fond des chambres (1, 2) et le tube de communication (6) à la base sont munis de filtres (7) ou de moyens aptes à empêcher l'entrée de poussière ou de débris à l'intérieur de l'appareil.

19. Dispositif selon la revendication 18, dans lequel lesdits filtres (7) peuvent être changés.

20. Dispositif selon l'une ou plusieurs des revendications 1 à 19, dans lequel ledit dispositif est de préférence fermé et comprend de préférence une surface rugueuse sur le côté opposé à celui avec le tuyau (5).
